Europäisches Patentamt

European Patent Office

Office européen des brevets

(11) Publication number: **0 227 258**
**A2**

# (12) EUROPEAN PATENT APPLICATION

(21) Application number: **86308339.0**

(22) Date of filing: **27.10.86**

(51) Int. Cl.⁴: **A 61 B 17/36**

(30) Priority: **08.11.85 GB 8527563**

(43) Date of publication of application:
**01.07.87 Bulletin 87/27**

(84) Designated Contracting States:
**AT BE CH DE ES FR GB GR IT LI LU NL SE**

(71) Applicant: **MICRA Ltd.**
**54 Alma Street**
**Luton Bedfordshire LU1 2PL(GB)**

(72) Inventor: **Hoskin, William John**
**5, Long Buftlers**
**Harpenden AL5 1JF(GB)**

(72) Inventor: **Pierse, Dermot John**
**143 Harley Street**
**London W1N 1DJ(GB)**

(74) Representative: **Enskat, Michael Antony Frank et al,**
**Saunders & Dolleymore 2 Norfolk Road**
**Rickmansworth Hertfordshire WD3 1JH(GB)**

(54) **Surgical cutting and piercing apparatus.**

(57) A surgical knife includes a handle (3) supporting a diamond blade (4). An Nd YAG laser (5) is optically coupled by a bundle of optical fibres (2) to the blade (4). The diamond blade (4) is so profiled as to focus the laser energy into a cutting edge-like configuration ahead of the diamond blade (4). The arrangement enables the laser energy to both cut and seal tissue being incised by the knife.

The focusing of the laser light by the diamond blade (4) provides the blade (4) with an unexpectedly increased cutting and cauterising performance and an increased life for the blade.

FIG.1

EP 0 227 258 A2

- 1 -

SURGICAL CUTTING AND PIERCING APPARATUS

The present invention relates to surgical cutting or piercing implements for coupling to a laser source.

Our corresponding European patent publication No. 0125897 describes surgical scalpels with diamond or sapphire blades which are heated by focussing the laser energy at the emission surface of the blade and by means of an additional heating element. The temperatures reached of around 500°C enable the efficient cutting of the tissue simultaneously with the cauterising of a tissue which contacts the heated surface of the blade or point. The disadvantage of this arrangement is that the high temperatures cause charring of the tissue and the such charred tissue tends to adhere to the blade surface reducing its cutting efficiency.

The same patent publication also describes a surgical probe with a diamond point used for the treatment of cancerous growths. With such an arrangement the laser beam is focused at the emission surface of the point to heat the point and additional heat is provided by a supplementary heater.

The point is then inserted into the centre of the cancerous growth and the heat generated from the point, which is at 500°C, kills the surrounding cells. The disadvantage of this arrangement is that the probe kills cancerous and healthy cells alike within a predetermined radius. Also tissue contacting the point tends to become charred and adheres to the point reducing the heat and part of the point and its piercing efficiency.

It is an object of the invention to provide an improved surgical implement.

According to the present invention there is provided a surgical implement comprising a source of laser energy and focusing means for focusing the laser energy into the configuration of a cutting edge located ahead of the leading edge of the knife.

According to the present invention there is further provided a surgical probe comprising a stiletto shaped member terminating in a focusing member, a source of laser energy and means for directing the laser energy from the source to the focusing member, through the stiletto shaped member, the focusing member being of such configuration as to direct the laser energy received into a generally part-spherical configuration about the focusing member so that when the stiletto shaped member is introduced into a patient with the focusing member located in a central portion of a cancerous tissue, laser energy is uniformly propagated from the focusing member into the tissue to destroy the cells thereof.

According to the present invention there is still further provided a method of supplying laser energy to a surgical cutting or piercing implement comprising the steps of directing the energy to a focusing member, arranging the focusing member to focus the energy beyond the surfaces of the focusing member, and maintaining the surface temperature of the focusing member at a level below 65°C.

Surgical implements embodying the invention will now be described, by way of example only, with reference to the accompanying diagrammatic drawings in which:-

Figure 1 is a plan view of a laser knife;

Figure 2 is a perspective view of another form of laser knife;

Figure 3 is a perspective view to an

2629EP
0227258

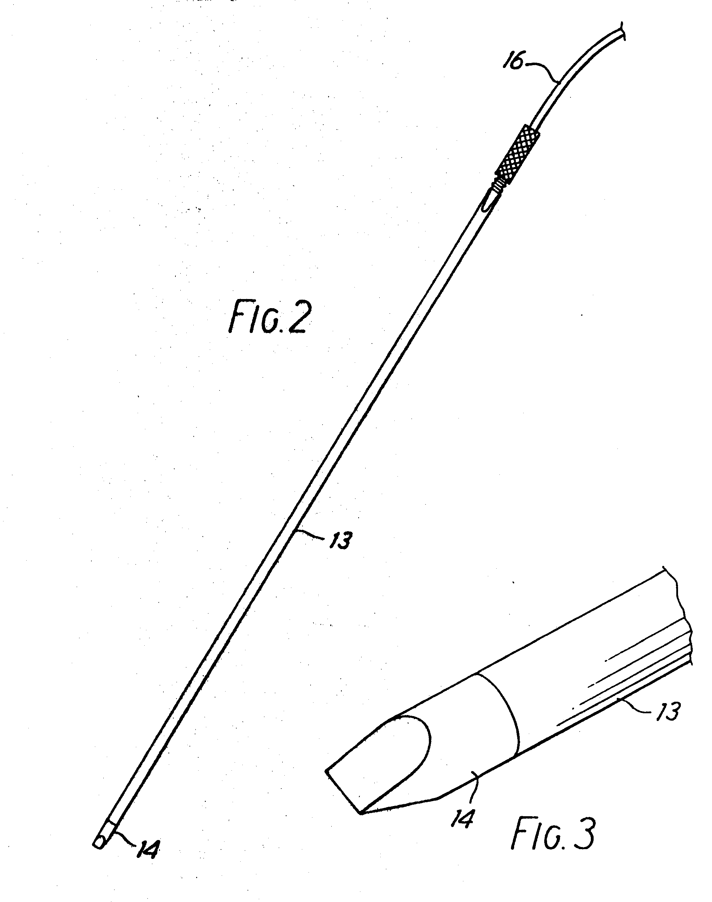

enlarged scale of the tip portion of the knife of Figure 2;

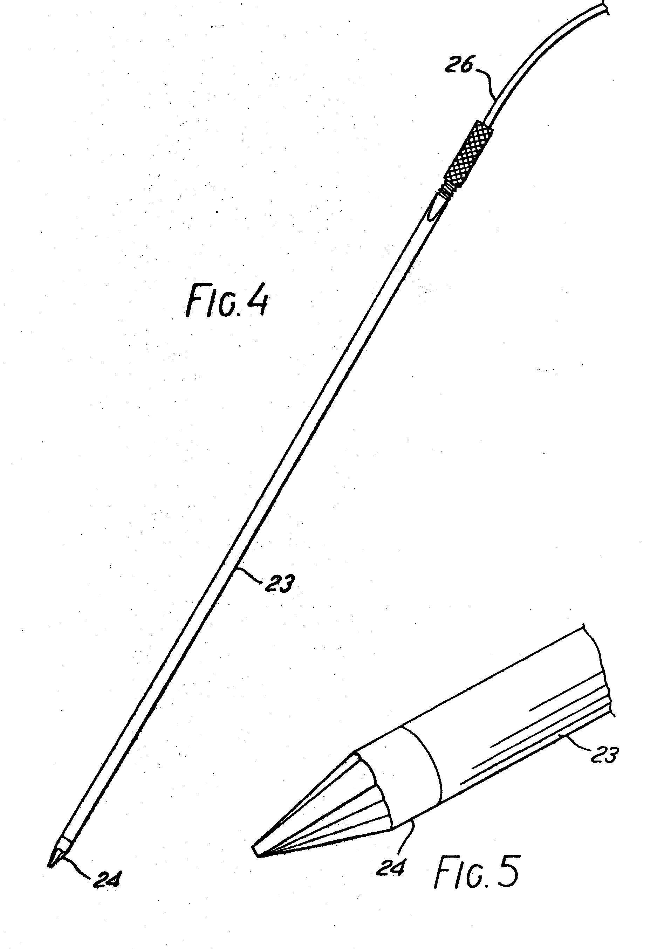

Figure 4 is a perspective view of yet another form of laser knife; and

Figure 5 is a perspective view to an enlarged scale of the tip portion of the knife of Figure 4.

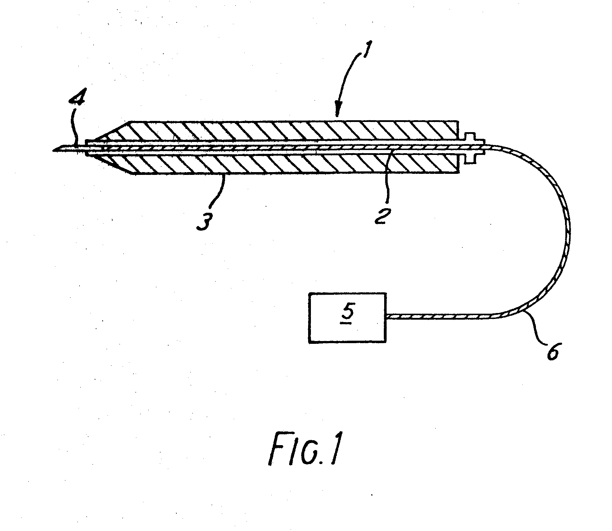

As shown in Figure 1 a surgical diamond knife 1 has a handle 3 supporting a diamond blade 4. An optical fibre bundle 2 located within the handle 3 abuts an optically smooth surface of the diamond blade 4. A connector 6 couples the other end of the optical fibre bundle 2 to a radiation source in the form of a Neodymium/Yttrium Aluminium Garnet (Nd/YAG) Laser 5.

The diamond blade is so profiled as to focus the laser energy into a cutting edge-like configuration to define a notional cutting edge beyond the edge of the diamond blade.

The Nd/YAG laser 5 is in the form of a crystal providing a continuous source of power.

The laser is operated continuously at a low power (e.g. 1 to 4 watts output).

With the above-described specific arrangement, it is the focused laser energy which provides a cutting action through the tissue and the resultant power dissipated into the cut surfaces then has two effects. Firstly, it provides a cauterising action to seal the cut surface and secondly the power causes the blood vessels, and other fluid conduits below the cut surfaces to contract and so restrict the flow of blood and other fluids in the region of the cut surfaces.

The combination of these effects allows the laser to be operated at an unexpectedly low power

2629EP
0227258

level to achieve a speedy seal of the incised tissue against fluid loss as the cutting action progresses.

The free flow of body fluids is thus prevented and the chances of patient recovery from a surgical operation are significantly improved.

The laser knife described may, optionally, be combined with a conventional visible light source to cause the blade to luminesce (as disclosed in our published British Patent Application No. 8123635). This combination is particularly useful when the knife is used, e.g. in brain surgery where most incisions are made through small holes which may be deep and therefore dark.

It will be appreciated that because of the focusing provision of the laser energy less power is needed to effect cauterisation and so instead of a diamond blade any other crystal blade (for example a sapphire blade) can be used to focus the laser energy into a cutting edge-like configuration.

The crystal blades used need not be sharp since the cutting action is effected by the laser cutting edge and so the blade surfaces may merely function to increase the spacing between the cut surfaces by pushing them further apart.

Indeed instead of crystals any other mechanism can be used which acts to focus laser energy into a cutting edge-like configuration. The need for sharp cutting edges can thus be obviated.

It has been discovered that the temperature for killing cancerous cells is generally less than that required to kill healthy cells. With one type of lung cancer for example it has been noted that a temperature 55°C will kill the cancer cells but not the surrounding healthy cells. Thus, if the energy from a Nd YAG laser can be directed into the centre of the tumour and the temperature of the tumour raised to at least a level of 55°C, then by sustaining this temperature for a period of time the cancerous cells will be killed and healthy cells generally will not be killed. Where temperatures higher than 55°C are used, care should be taken to ensure that the temperature gradient through the tumour is such that beyond the boundaries of the tumour the normal cells are subjected to a temperature of no greater than 55°C to avoid the destruction of such normal cells. Care must also be taken not to raise the upper limit of the temperature beyond a level at which carbonisation of the cells occurs.

This treatment can be effected under local anaesthetic by using stiletto shaped diamond probes which can be directly pushed through the wall of the chest into the centre of the tumour. In this case the facets of the diamond point should be such that the laser energy is distributed in part-spherical configuration so that the laser energy can be dissipated generally uniformly throughout the tumour Sustained dissipation of energy into the cancer cells will eventually destroy them.

The advantage of such treatment is that the resultant debris within the patient's chest can be coughed up by the patient leaving a clean scar and because only a local anaesthetic is required, the treatment can be conducted in environments outside an operating theatre, for example, in an out patient department.

Two different forms of probe particularly suitable for such treatment of cancerous tissue are shown in Figures 2 and 3 and Figures 4 and 5.

The probe shown in Figures 2 and 3 has a handle 13 supporting a diamond point 14. The handle 13 houses an optical fibre bundle (not shown) which is coupled at one end to the point 14 and to a connector 16 at the other end.

The diamond point 14 as shown more clearly in Figure 3 has a cylindrical body portion terminating in a wedge-shaped tip. The leading edge of the tip has a length substantially equal to the diameter of the cylindrical body portion.

The probe shown in Figures 4 and 5 has a handle 23 supporting a diamond point 24. The handle 23 houses an optical fibre bundle (not shown) which is coupled at one end to the blade 24 and to a connector 26 at the other end.

The diamond point 24 as shown in Figure 5 has a cylindrical body portion and a profiled tip portion. The profiled tip portion has an axially extending central portion which is wedge-shaped and two axially extending lateral portions each of which defines part of a multi-sided cone. The leading edge of the wedge of the central portion extends between 1/4 and 1/5 of the diameter of the body portion.

The probes of Figures 2 to 5 when operated for some 15 minutes or so using a low power laser (1-4 watts output) may acquire surface temperatures of 60-65°C. However, provided the healthy cells are located some distance from the surface they will not suffer adversely from the probe temperature.

In operation, the blade 24 will develop a mushroom shaped radiation pattern.

It will be appreciated that when the probes are used to destroy cancerous tumours the radiation pattern provided by the blade will determine the depth to which the blade needs to be pushed into the tumour.

2629FP

0227258

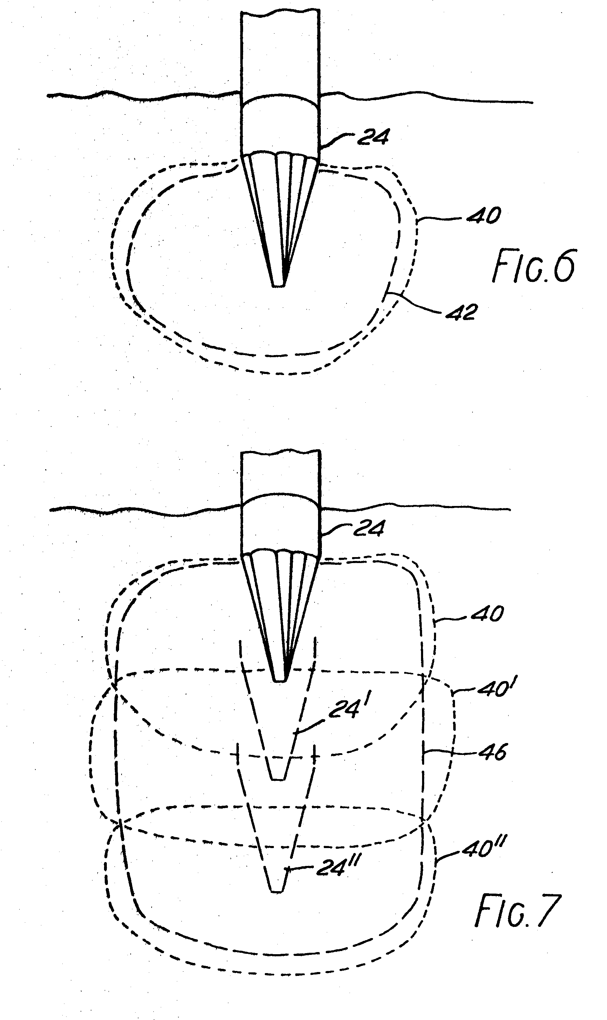

For example, to treat a mushroom shaped tumour 42 (see Fig. 6), the probe 24 of Figure 5 which provides a corresponding mushroom shaped pattern 40 needs only to be pushed a short way into the tumour.

If, however, the tumour 46 is cylindrical (see Fig. 7), then the tumour 46 can be treated in successive stages using the same probe 24 by progressively stepping the probe 24 deeper into the tumour along the axis of the tumour 46.

Thus, as shown in Figure 7, the tumour 46 is treated in three steps using the three successive positions 24, 24' and 24" of the blade 24 to create three overlapping patterns 40, 40' and 40" to cover the whole volume of the tumour.

With different profiles of tumour, probes providing correspondingly shaped radiation patterns can be used. If the tumour is of irregular shape, the skill of the surgeon will determine which probe to use, the positions it must take up and the period that it is subjected to radiation.

Under certain conditions one or more facets of the probe may be shielded by coating with a gold layer to direct the radiation to one side of the blade. Such probes would be particularly useful for treating concerous tissue around the throat of the patient where it is particularly important to avoid destroying the wall of the throat while at the same time subjecting the cancerous tissue adjacent the wall to the full extent of the laser radiation.

In a modification radiation from the laser may be coupled from the laser to the diamond implement by means of alternative methods such as those disclosed in co-pending Application No. 81 23635 (Published Specification No. 2 102 678).

- 8 -

0227258

It will thus be seen that the operational temperatures of the blades and points of the surgical implements disclosed herein are considerably lower than the operational temperatures of the prior art.

It has thus been found that significant benefits accrue from operating the blades and points at temperatures of less than 65°C.

CLAIMS

1.      A surgical implement comprising a source of laser energy and focusing means for focusing the laser energy into the configuration of a cutting edge located ahead of the leading portion of the implement.

2.      A surgical implement according to Claim 1 wherein the laser comprises a Neodymium/Yttrium Aluminium Garnet laser.

3.      A surgical implement according to Claim 1 or to Claim 2 wherein the focusing means comprises a diamond cutting edge.

4.      A surgical implement according to any one of Claims 1 to 3 wherein the source of laser energy is set to run at an energy level in the range of from 1 to 4 watts.

5.      A surgical knife comprising a transparent blade, a Neodymium/Yttrium Aluminium Garnet laser, and means optically coupling the radiation output from the laser to the vicinity of the cutting edge of the blade, to enable the blade to focus the laser energy into a cutting edge-like configuration located ahead of the blade to both cut and cauterise tissue being incised by the knife.

6.      A knife according to Claim 5 wherein the optically coupling means comprises a bundle of optical fibres extending from an optically smooth surface of the transparent blade to the laser.

7.      A knife according to Claim 6 including a handle supporting the blade and through which said bundle of optical fibres pass.

8.      A knife according to any one of Claims 5 to 7 including the combination therewith of a source of visible light which is transmitted to the blade to illuminate the area of the incision.

2629EP

0227258

9.      A surgical implement comprising a stiletto shaped member terminating in a focusing member, a source of laser energy and means for directing the laser energy from the source to the focusing member, through the stiletto shaped member, the focusing member being of such configuration as to direct the laser energy received into a generally part-spherical configuration about the focusing member so that when the stiletto shaped member is introduced into a patient with the focusing member located in a central portion of a cancerous tissue, laser energy is uniformly propagated from the focusing member into the tissue to destroy the cells thereof.

10.      An implement according to Claim 9 wherein the focusing member comprises a diamond tip.

11.      An implement according to any preceding claim wherein the focusing member comprises a cylindrical body portion and a wedge-shaped tip portion.

12.      An implement according to Claim 11 wherein the leading edge of the tip portion is substantially equal in length to the diameter of the body portion.

13.      An implement according to Claim 11 wherein the length of the leading edge of the tip portion is substantially in the range of from 1/4 to 1/5 of the diameter of the body portion.

14.      A method of supplying laser energy to a surgical cutting or piercing implement comprising the steps of directing the energy to a focusing member, arranging the focusing member to focus the energy beyond the surfaces of the focusing member, and maintaining the surface temperature of the focusing member at a level below 65°C.

FIG.1

FIG.2

FIG.3

FIG.4

26

23

24

FIG.5

23

24

0227258

*FIG.6*

*FIG.7*